# EUROPEAN PATENT SPECIFICATION

(11) **EP 1 600 498 B1**
(45) Date of publication and mention of the grant of the patent: **15.08.2012**
(21) Application number: 03703212.5
(22) Date of filing: 06.02.2003
(51) Int. Cl.: G01N 33/50

(54) **SUBSTRATES FOR HIGH-DENSITY CELL ARRAYS AND PREPARATION PROCESSES AND USES THEREOF**
SUBSTRATEN FÜR HOCHDICHTE-ZELLANORDNUNGSLEITERPLATTE, PROZESS ZUM HERSTELLEN UND VERFAHREN ZUM VERWENDEN DERSELBEN
SUBSTATS POUR PLATEAUX A RESEAUX DE CELLULES HAUTE DENSITE, PROCEDE DE PRODUCTION ET SON UTILISATION

(43) Date of publication of application: 30.11.2005
(73) Proprietor: Cellseed Inc., Tokyo 162-0056 (JP)
(72) Inventor: YAMATO, Masayuki, Setagaya-ku, Tokyo 158-0097 (JP); OKANO, Teruo, Ichikawa-shi, Chiba 272-0827 (JP)
(74) Representative: HOFFMANN EITLE
(86) International application number: PCT/JP2003/001247
(87) International publication number: WO 2004/069988

(56) References cited:
- WO-A-01/07891
- WO-A-02/089982
- WO-A2-02/59603
- US-A1- 2002 057 995
- US-A1- 2002 132 272
- DATABASE BIOSIS [Online] BARSKII B.E. ET AL.: 'Biological microchips with hydrogel-immobilized nucleic acids, proteins and other compounds: properties and applications in genomics', XP002965889 Database accession no. 200200495902 & MOLEKULYARNAYA BIOLOGIYA vol. 36, no. 4, July 2002 - August 2002, MOSCOW, pages 563 - 584
- NICOLAU D.V. ET AL.: 'Patterning neuronal and glia cells on light-assisted functionalised photoresists' BIOSENS. BIOELECTRON. vol. 14, no. 3, 1999, pages 317 - 325, XP002965890
- KAM L.: 'Correlation of astroglial cell function on micro-patterned surfaces with specific geometric parameters' BIOMATERIALS vol. 20, no. 23-24, 1999, pages 2343 - 2350, XP002965891
- YAMATO M. ET AL.: 'Novel patterned cell co-culture utilizing thermally responsive grafted polymer surfaces' J. BIOMED. MATER. RES. vol. 55, no. 1, April 2001, pages 137 - 140, XP002965892
- DATABASE MEDLINE [Online] HIROSE M. ET AL.: 'Creation of designed shape cell sheets that are noninvasively harvested and moved onto another surface', XP002965893 Database accession no. 21567368 & BIOMACROMOLECULES vol. 1, no. 3, 2000, pages 377 - 381
- YAMATO M. ET AL.: 'Thermally responsive polymer-grafted surfaces facilitate patterned cell seeding and co-culture' BIOMATERIALS vol. 23, no. 2, January 2002, pages 561 - 567, XP004348063

## Description

### TECHNICAL FIELD

The present invention relates to a novel culture substrate, a substrate for high-density cell arrays using said culture substrate and a process for preparing them. The present invention also relates to methods for using each cell on the substrate for high-density cell arrays from which culture medium is removed in the evaluations of chemicals or the like and in gene transfer processes or the like. Moreover, the present invention relates to methods for separating and recovering only cells having a specific activity after the cell evaluations or genetic manipulations from the substrate. Furthermore, the present invention relates to screening methods of chemicals such as medicines, toxic agents and so on and gene transfer methods using the methods of the invention.

### BACKGROUND ART

Drug therapy for cancers yielded very significant achievements in treating cancers of blood cells or the like, but has not yet yielded sufficient achievements in treating some types of solid cancers. One of the reasons for the lack of success is that the effects of each drug vary between cancer cells, and moreover, there is no effective means for evaluating, before actual administration, the efficaciousness and the required dosage of anticancer agents. In view of a large variety of anticancer agents with varying formulae, the development of a system capable of conveniently assaying multiple anticancer agents using cultured cells would greatly promote the current cancer drug therapy that has not yet sufficiently avoided adverse side effects because such a system could determine the efficaciousness or necessary dosage of multiple anticancer agents on cultured cancer cells collected from tumors before they are actually prescribed for patients. However, clinical application of such a system is definitely impractical in terms of both scale and necessary labor because the number of cells that can be obtained by biopsy are too small to assay multiple formulae by conventional culture methods, which also require culture vessels as many as multiple analytes. In recent years, the development of techniques for evaluating drugs/toxic agents using cultured cells from humans or the like has received a great deal of attention, especially in view of the scarcity of experimental animals for reasons of low reproducibility in the animals, and due to individual difference and species differences between animals and humans. The need to develop techniques for rapidly and simultaneously assaying multiple drugs/toxic agents has also been strongly emphasized from the viewpoints of cost reduction of new drug development, safety testing of medicines, environmental impact assessment, and others. To meet these needs, attention has been focused on the development of a device for performing electrophoresis and chromatography on a substrate having a size of about several centimeters to 10 cm by applying microfabrication techniques known as semiconductor processing techniques ("Lab-on-Chip"). For the electrophoresis of nucleic acids, for example, fully automated systems have already been commercialized, which are capable of automatically performing all the steps such as gel preparation, electrophoresis, band detection and data analysis that was conventionally performed in separate devices. The "Lab-on-Chip" technique can be used to provide the following advantages: (1) only small amounts of samples are required; (2) multiple analytes can be simultaneously assayed in parallel on a large scale; (3) high reproducibility is achieved because manual operations can be automated by systemization; (4) influences on the environment can be minimized because waste liquid dramatically decreases; (5) high safety is achieved by drastically decreasing the amounts of toxic reagents used; (6) analytical costs can be reduced.

It is desirable to assay antibodies and enzymes by microanalyses and quantification based on their high specificity, but conventional methods had to rely on experimental animals to understand reactions shown by living bodies. Cells are regarded as being extremely well suited because of their potential use for measuring the quality of influences of drugs/toxic agents of interest on living bodies rather than simply measuring the amounts because even a single cell contains complex information processing powers and chemical reactions comparable to computers or chemical complexes. Existing systems for measuring single cell units include flow cytometers, which have greatly contributed to the progress of immunology by achieving quantitative measurements of membrane surface antigens, for example, but already have a history of 20 years or more and it does not appear that they will make any further significant progress in the future. Flow cytometers are designed to assay suspended cells such as blood cells and require adhesive cells cultured on culture dishes to be recovered from the culture dishes and suspended, and they cannot be used for simultaneous super-multianalyte evaluations of adhesive cancer cells, for example. Recently, devices called laser scanning cytometry combining a computer-controlled X-Y stage and a fluorescent microscope have been commercially available. These devices are designed to measure each cell in a tissue sample on a slide glass. Thus, it is difficult to use them to simultaneously evaluate a number of different drugs under different conditions because all cells on one preparation are subjected to the same conditions but drug evaluations require as many preparations as the number of drugs and conditions.

WO0107891 discloses arrays for cell screening, and methods for making them, comprising a substrate with three dimensional hydrated polymer constructs in the reaction wells, wherein the hydrated polymer suspension comprises cell adhesion promoters and cells to be analyzed, and wherein the substrate further comprises inter-well spaces comprising one or more cell adhesion inhibitors.

We were keenly aware of the importance of the problem above and devoted to the research and development of a system for simultaneously assaying multiple analytes. As a result, we succeeded in preparing a culture substrate for a high-density cell array on which cells can be cultured at such a high density as 500-100,000 cells per cm² in a culture medium completely separated for each cell by using materials having different affinities to water. We also prepared an automatic chemical dispenser (nanodispenser) for automatically dispensing various chemicals at various concentrations by adapting inkjet printer heads and completed a system capable of simultaneously assaying multiple analytes only by using a single culture substrate for a high-density cell array. Moreover, we achieved a technique capable of separating and recovering only specific cells on the substrate after an assay has been finished and directing the cells for further purposes. The present invention was attained on the basis of these findings.

### DISCLOSURE OF THE INVENTION

The present invention provides a substrate for a high-density cell array, having a novel surface comprising an array of high-density domains coated with a cell adhesive polymer and successively surrounded by a domain coated with a cell non-adhesive hydrophilic polymer and then a domain coated with a cell non-adhesive highly hydrophobic material. The present invention also provides a process for preparing the substrate for a high-density cell array, comprising subjecting a stack of at least three materials on a base to laser ablation in such a manner that each layer partially appears as a part of the surface of the substrate. In addition, the present invention provides a method for using each cell in a separated culture medium on the substrate for a high-density cell array in the evaluation of a chemical and a gene transfer process or the like. Moreover, the present invention provides a method for separating and recovering only cells which have a specific activity after a cell evaluation or genetic manipulation is carried out. Furthermore, the present invention provides a screening method for a chemical such as a medicine or a toxic agent and a gene transfer method, comprising using said high-density cell array.

### BRIEF DESCRIPTION OF THE DRAWINGS

Figure 1 shows an example of a high-density cell array substrate of the present invention.
Figure 2 is a flowchart showing a process for preparing the high-density cell array substrate of the present invention.
Figure 3 (a) shows a part of a high-density cell array substrate on which cells and a culture medium have been arranged. Figure 3 (b) shows an example of a high-density cell array substrate of the present invention.
Figure 4 illustrates a multianalyte cell assay method using an inkjet printer according to the present invention.
Figure 5 shows hepatic cells arranged on a high-density cell array substrate of the present invention.

### THE MOST PREFERRED EMBODIMENTS OF THE INVENTION

The terms used herein in relation to the present invention are explained below.

Cell array: Attention is being given to DNA arrays on which a number of DNA fragments (oligonucleotides) have been immobilized for the purpose of large scale parallel assays. The term array originally has a meaning synonymous to "alignment", but is sometimes also referred to as a DNA chip because some DNA arrays are prepared by photolithography as used for preparing semiconductors. As an extension of the DNA array technique, protein arrays in which a number of antibodies are arranged have been developed. Cell arrays in which cells are arranged at a high density are considered as a next-generation technique following DNA arrays and protein arrays. In the present invention, a single cell is adhered to a domain coated with a cell adhesive polymer to arrange 500-100,000 cells on a substrate for a high-density cell array for the purpose of parallel multianalyte assays.

Nanodispenser: On cell arrays of the present invention, 500-100,000 culture conditions can be simultaneously assayed because each cell is cultured in a completely separate culture medium. A device capable of dispensing a specified amount of a chemical is called a dispenser, and herein called nanodispenser, which means that a drug solution on the nano- to micro-liter scale is injected into a culture medium within each domain.

High-throughput screening: Conventional methods for new drug development or evaluation of a toxic agent had to use a lot of culture dishes and experimental animals, which resulted in enormous screening costs required for searching active drugs. With the recent development of combinatorial chemistry technology, the number of substances to be evaluated has dramatically increased and the importance of inexpensive and rapid evaluations and searches become increasingly emphasized, thus leading to strong demands for "high-throughput screening" as a new super-parallel technology.

Inkjet printer head: Many commercially available color inkjet printer heads are disposable and cost about 1,000 Japanese yen each. Such inexpensive heads can dispense several picoliters of liquid drops with sufficient precision. The system of the present invention also uses disposable heads for each drug, whereby the possibility of contamination and maintenance can be minimized.

Confocal laser scanning microscope: Fluorescence can be detected with high precision and good reproducibility by using a laser beam as a light source and a photomultiplier as a detector. Laser light is the most suitable for the system of the present invention because it scans only a necessary range on a focal plane using a galvanometer mirror so that it retains colors even during observation on large areas.

Lab-on-Chip: A new technology for performing electrophoresis and PCR and other operations on a single chip on which reaction layers and flow channels or the like have been prepared by microfabrication techniques. This can reduce the amounts of drugs required and increase speed.

Electron beam-induced polymerization: Radical polymerization using radicals generated by electron beam irradiation. This allows covalent immobilization on large areas. This method has been already widely applied in industries and can be performed at low cost.

Laser ablation: A technique for microfabricating a surface by irradiating it with a laser to break the bonds between molecules constituting the surface via the energy of the laser.

There have been a few priliminary studies reporting techniques for arranging a number of cells on a substrate by preparing cell adhesive domains of a predetermined size each containing a single cell. In principle, photolithography technique is used with silicon or glass as a base material. However, these techniques are not suitable for mass production, and all reports show that only about several tens to one hundred cells are arranged in a domain in the order of millimeters. In contrast to this, the present invention uses inexpensive plastic materials rather than silicon or glass to prepare substrates and adopts processing techniques suitable for mass production, thus promising to ensure considerable cost reduction. The electron beam-induced polymerization and laser ablation techniques used in the present invention are readily adaptable for processing large areas, i.e. centimeter-scale high-density substrates can be readily prepared. Yamato M., Kwon O.H., Hirose M., Kikuchi A., Okano T., "Novel patterned cell co-culture utilizing thermally responsive grafted polymer surfaces", J. Biomedical Material Research, 55, 137(2001) describes that patterned co-culture of multiple types of cells was achieved at unprecedentedly low costs and convenience by immobilizing a polymer in a pattern on the surface of a culture dish via the electron beam irradiation technique as used in the present invention. Hirose H., Kwon O.H., Yamato M., Kikuchi A., Okano T., "Creation of designed cell sheets that are noninvasively harvested and moved to another surface", Biomacromolecules, 1, 377-381(2000) describes that two polymers are immobilized on the same surface to prepare a cell adhesive weakly hydrophobic domain and a cell non-adhesive highly hydrophilic domain. Both documents show constituent techniques of the present invention.

As used herein, "high density" means a surface on which 500-100,000 domains coated with a cell adhesive polymer can be discretely arranged per cm² of a culture substrate. Preferably, the culture substrate has a surface on which 1,000-50,000, especially 10,000-30,000 domains can be discretely arranged per cm². In the present invention, cells are adhered to each of the cell adhesive domains with no limitation on the number of cells adhered to, but typically 1-20 cells, preferably 1-5 cells, more preferably 1-2 cells should be adhered to each cell adhesive domain in view of the size of the cell adhesive domain and the size of cells. The cell adhesive polymer of the present invention is not specifically limited, but may be, for example, a polymer having an ionic group, hydrophilic/hydrophobic group or the like optionally surface-treated by glow discharge, corona discharge or the like after it is coated on a base surface, or selected from any one of cell adhesive proteins such as fibronectin, collagen, laminin or combinations thereof optionally treated.

In the present invention, a stimulus-responsive polymer is used as the cell adhesive polymer, whereby only cells having a specific activity can be selectively separated after an assay of a chemical or gene transfer. An example of the stimulus-responsive polymer is a temperature-responsive polymer, which may be a homopolymer or copolymer. Basic building blocks of temperature-responsive polymers that can be used include, for example, (meth)acrylamide compounds such as acrylamides and methacrylamides; N-alkyl-substituted (meth)acrylamide derivatives such as N-ethyl acrylamide (lower critical solution temperature of homopolymer 72°C; the values within the parentheses below have the same meaning), N-n-propyl acrylamide (21°C), N-n-propyl methacrylamide (27°C), N-isopropyl acrylamide (32°C), N-isopropyl methacrylamide (43°C), N-cyclopropyl acrylamide (45°C), N-cyclopropyl methacrylamide (60°C), N-ethoxyethyl acrylamide (about 35°C), N-ethoxyethyl methacrylamide (about 45°C), N-tetrahydrofurfuryl acrylamide (about 28°C), N-tetrahydrofurfuryl methacrylamide (about 35°C); N,N-dialkyl-substituted (meth)acrylamide derivatives such as N,N-dimethyl (meth)acrylamide, N,N-ethylmethyl acrylamide (56°C), N,N-diethyl acrylamide (32°C); (meth)acrylamide derivatives having a cyclic group such as 1-(1-oxo-2-propenyl)-pyrrolidine (56°C), 1-(1-oxo-2-propenyl)-piperidine (about 6°C), 4-(1-oxo-2-propenyl)-morpholine, 1-(1-oxo-2-methyl-2-propenyl)-pyrrolidine, 1-(1-oxo-2-methyl-2-propenyl)-piperidine, 4-(1-oxo-2-methyl-2-propenyl)-morpholine; and vinyl ether derivatives such as methyl vinyl ether (lower critical solution temperature of homopolymer 35°C). Copolymers with monomers other than those mentioned above, graft polymerization products or copolymerization products of the polymers, or mixtures of the copolymers or polymers may also be used when the critical solution temperature should be controlled depending on the types of cells or when the interaction between the coating material and the base should be enhanced or for the purpose of controlling the hydrophilic/hydrophobic balance of the cell adhesive surface or other purposes. The polymers may be crosslinked so far as their intrinsic properties are not affected. Another example of the stimulus-responsive polymer is a photoresponsive polymer including polymers inducing photoisomerization reactions such as absorptive polymers having an azobenzene group, temperature-responsive polymers having a functional group capable of photoion dissociation such as copolymers of vinyl derivatives of triphenylmethane leucohydroxide and acrylamide monomers, or temperature-responsive polymers associated with light-dependent hydrophobic interaction such as N-isopropylacrylamide gel containing spirobenzopyran. The base to be coated can be made from not only materials normally used for cell culture such as glass, modified glass, polystyrene, polymethyl methacrylate, but also any material capable of being shaped such as polymer compounds other than those mentioned above, ceramics and metals. It may also be in the form of a plate, but not limited to a cell culture dish such as a Petri dish.

The base can be coated with a cell adhesive polymer by either (1) bonding the base and the coating material via chemical reactions, or (2) using physical interactions or a combination of both methods. Thus, (1) bonding via chemical reactions can be accomplished by using electron beam irradiation (electron beam; EB), γ irradiation, UV irradiation, plasma treatment, corona treatment, etc. Moreover, conventional organic reactions such as radical reaction, anionic reaction and cationic reaction can be used when the base and the coating material have suitable reactive functional groups. (2) Bonding via physical interactions can be accomplished by physical adsorption such as coating or kneading of the coating material alone or in combination with a medium consisting of a matrix compatible with the base (e.g., graft polymers, block polymers or the like of the monomer forming the base or a monomer compatible with the base and the coating material). When stimulus-responsive polymers are not selected as cell adhesive polymers, the coating amount is not limited, but when stimulus-responsive polymers are selected as cell adhesive polymers, the coating amount should be in the range of 0.1-5.0 µg/cm², preferably 0.3-3.0 µg/cm², more preferably 0.5-2.5 µg/cm². The coating amount of less than 0.1 µg/cm² is not preferred because cells on such polymers are difficult to separate even under stimulation and the working efficiency is considerably lowered. If it is 5.0 µg/cm² or more, however, cells cannot readily adhere to such domains, whereby they become difficult to sufficiently adhere. The coating amount of the polymers can be determined by using the following techniques alone or in combination, such as Fourier Transform Infrared Attenuated Total Reflection (FT-IR-ATR), analyses by staining of coated domains or staining of fluorescent substances, or surface analyses using contact angle measurements or the like. When the cell adhesive domain of the present invention is smaller than the analyzable range, the coating amount should be preliminarily determined in the analyzable range and may be applied under the same conditions.

The high-density cell array substrate of the present invention comprises domains coated with a cell adhesive polymer as described above and surrounded by a domain coated with a cell non-adhesive hydrophilic polymer. The number of the domains coated with a cell adhesive polymer within the domain coated with a cell non-adhesive hydrophilic polymer is not limited, but preferably 1-50, more preferably 1-20, especially 1-5. In the high-density cell array substrate of the present invention, the culture medium is divided among domains coated with a cell non-adhesive hydrophilic polymer, and therefore, the number of domains coated with a cell adhesive polymer within a domain coated with a cell non-adhesive hydrophilic polymer is preferably smaller because more cells can be cultured under different conditions and therefore, more analytes can be assayed. The cell non-adhesive hydrophilic polymer may be any polymer without limitation that resists cell adhesion and has high affinity for water, including e.g. polyacrylamide, polydimethyl acrylamide, polyethylene glycol, cellulose, etc. The coating amount of the cell non-adhesive hydrophilic polymer is not limited so far as it is enough to retain a small amount of culture medium, but should be in the range of 0.1-20.0 µg/cm², preferably 0.3-10.0 µg/cm², more preferably 0.5-5.0 µg/cm². The coating amount of less than 0.1 µg/cm² is not preferred because a small amount of culture medium is difficult to retain, but the amount of 20.0 µg/cm² or more is not preferred because such a domain shows significant irregularities during microscopic observation. Each analytical value shown here can be determined by the method described above. The cell non-adhesive hydrophilic polymer can be immobilized on the base by the method as described for the cell adhesive polymer.

In the present invention, the domain coated with a cell non-adhesive hydrophilic polymer is further surrounded by a domain coated with a cell non-adhesive highly hydrophobic material. The material may be any material without limitation that resists cell adhesion and repels water, including e.g. silicone polymers and derivatives thereof, fluorine-containing polymers and derivatives thereof, etc. The coating amount of the cell non-adhesive highly hydrophobic material is not limited so far as it is enough to repel a small amount of culture medium, but should be in the range of 0.1-20.0 µg/cm², preferably 0.3-10.0 µg/cm², more preferably 0.5-5.0 µg/cm². The coating amount of less than 0.1 µg/cm² is not preferred because a small amount of culture medium is difficult to repel, but the amount of 20.0 µg/cm² or more is not preferred because such a domain shows significant irregularities during microscopic observation. Each analytical value shown here can be determined by the method described above. The cell non-adhesive highly hydrophobic polymer can be immobilized on the base by the method as described for the cell adhesive polymer.

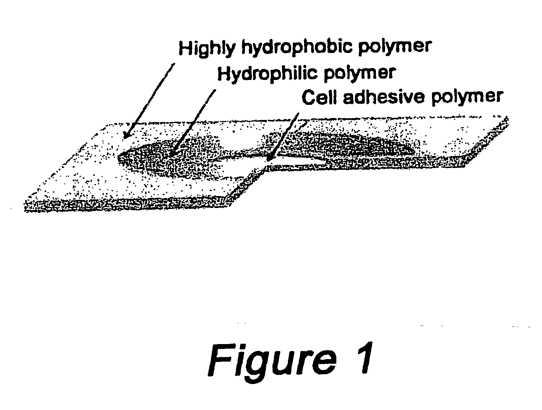

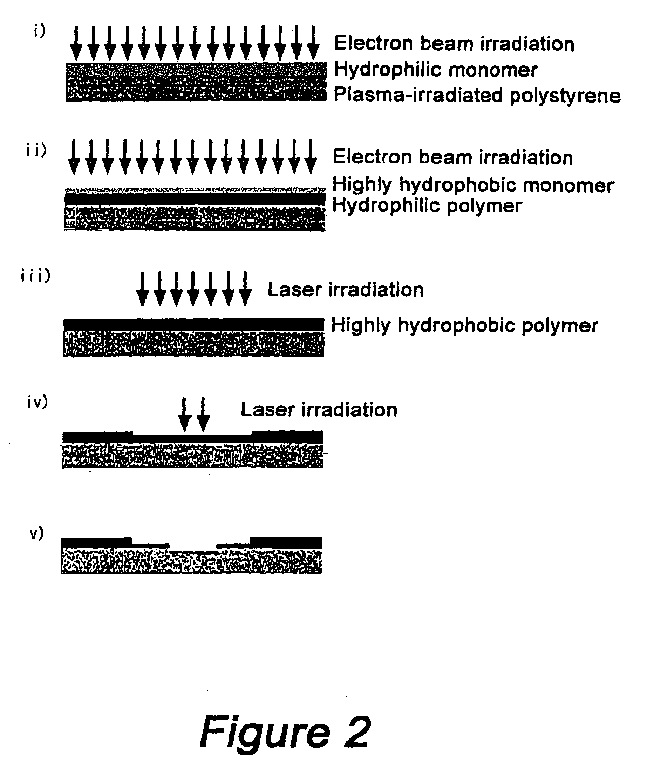

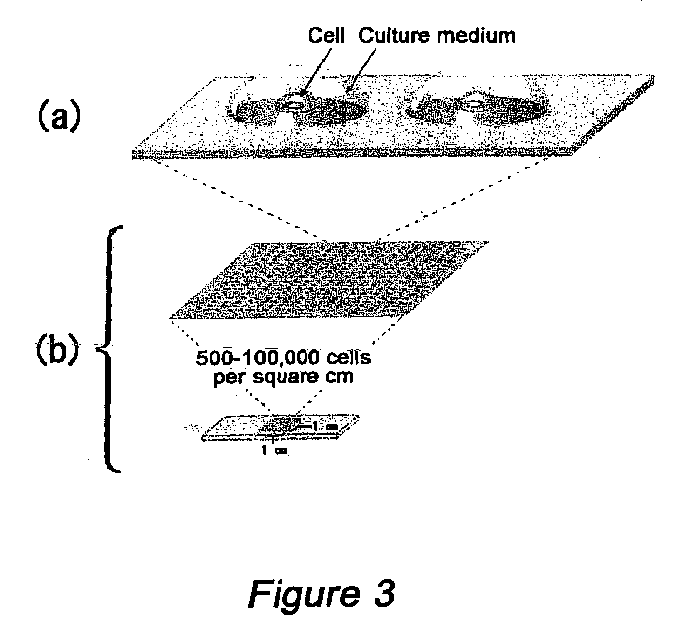

The process for preparing the high-density cell array substrate of the present invention may be any method without limitation that can form at least a domain capable of cell attachment surrounded by a domain capable of retaining a small amount of culture medium further surrounded by a domain capable of repelling a small amount of culture medium on the surface of the resulting high-density cell array substrate, including e.g. a process comprising providing a stack of a cell adhesive polymer layer, a hydrophilic polymer layer and a highly hydrophobic material layer in this order from the base side and cutting it by laser ablation in such a manner that each layer partially appears on the surface of the substrate. The resulting structure is shown in Figure 1. The process for preparing it is shown in Figure 2. Thus, polystyrene is used as a base and subjected to plasma irradiation to form a cell adhesive domain to which cells can adhere. On this polystyrene base, both hydrophilic and highly hydrophobic polymers are successively stacked by electron beam irradiation, after which the thickness of only one layer and two layers are cut by changing the power of a UV excimer laser to expose a hydrophilic domain and a cell adhesive domain. By this process, a cell adhesive domain having sufficient size for receiving a single cell (about 5-40 µm square) can be exposed. By repeating this operation, a cell array having a density of 500-100,000 cells/cm² can be prepared (Figure 3).

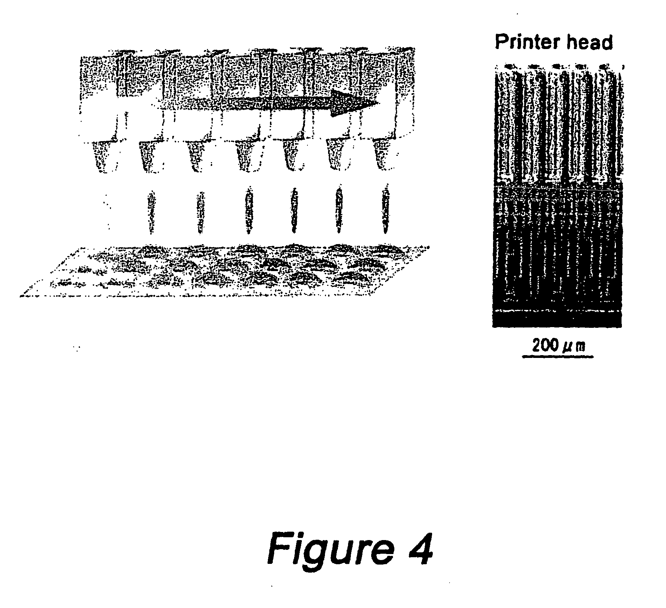

Onto the high-density cell array substrate thus obtained, cells are adhered and a culture medium is deposited independently in each hydrophilic polymer-coated domain by the method described below. First, cells can be adhered to the high-density cell array substrate only by dispersing given cells in a culture medium in an amount enough to cover the whole substrate and allowing them to stand in the conventional manner. The dispersed cells settle and adhere to only the cell adhesive domains. Then, excess culture medium is continuously and gently aspirated, whereby the culture medium can be left independently in each hydrophilic polymer-coated domain. The culture medium may be further aspirated and redeposited as small amounts of droplets to each cell using an inkjet printer head. A chemical (e.g., a drug, a toxic agent, etc.) at a given concentration can be automatically injected into the culture medium containing each cell on the high-density cell array substrate by using an automatic dispenser (nanodispenser) based on adapted inkjet printer heads having the function of automatically diluting chemicals as shown in Figure 4. According to this apparatus, an automatic dilution circuit consisting of a tank for storing chemicals, a tank for storing culture media for dilution, a micropump and a microvalve is connected to printer head channels, and the pump, valve and heads are controlled by a computer so that a drug can be delivered at any concentration. This apparatus was already shown to deliver convenient outputs of fluid volume corresponding to droplets on the order of two to several tens of picoliters. This is placed right above the cell array mounted on a computer-controlled X-Y stage and the drug is dispensed while the cell array is moved.

An assay system can be established by combining said high-density cell array substrate and said automatic dispenser with an existing confocal laser scanning microscope, a computer-controlled X-Y stage, and a fluorescent analyzer for detecting cell viability and cell activity.

Advantages of the technique of the present invention are specifically described below. The base for the cell array can be formed from plasma-treated polystyrene, which is inexpensive and suitable for observation under an optical microscope because of the transparency. A stimulus-responsive polymer, a hydrophilic polymer forming a hydrophilic domain necessary for retaining a culture medium for each cell, and a hydrophobic polymer forming a hydrophobic domain necessary for isolating the culture medium for each cell are successively stacked and covalently immobilized on the base. The immobilization of these layers begins with developing the lowermost monomer followed by electron beam irradiation and this operation is repeated until completion. This stack is ablated in any size and configuration using a UV laser, thereby preparing a substrate comprising a high-density array of cell adhesive polymer-coated domains (500-100,000 cells per cm²). Drugs can be automatically injected at any concentration into the culture medium containing each cell by using a nanodispenser with automatic dilution function based on adapted inkjet printer heads. A throughput of 500-100,000 cells is achieved within 1 minute without washing channels. Washing can be done for 10 seconds to 60 seconds per washing at each change of the dilution series, i.e. at each change of the drug. Cell reactions are detected by an existing confocal laser scanning microscope in which the X-Y stage is replaced by a computer-controlled one. It can also be connected to TOF-SIMS to detect all the proteins expressed in a single cell. For example, multiple drugs can be simultaneously assayed for concentration-dependent ability of killing cancer cells or anticancer cytotoxicity on a single cell array by using a fluorescent substance capable of detecting cell viability and cell activity after tens of thousands each of cancer and normal cells are seeded on the single cell array and subjected to the action of the drugs. This allows an automated super-multianalyte cell assay system to be inexpensively constructed. This system can be used for not only screening anticancer agents but also new drug development and environmental impact assessment. Moreover, the technique of the present invention allows only cells expressing a gene to be selectively recovered from the surface of an array substrate after the gene is transferred into a high-density array of cells or allows only cells showing a specific reaction to be selectively recovered from the surface of an array substrate after assaying cell activity against a chemical. The recovered cells can be grown and used as tissues or used as cell therapy agents or medical tools.

There has existed no report on a culture system capable of culturing human and animal cells in a culture medium compartmentalized into domains each containing a single cell to a few cells for the purpose of assaying drugs or toxic agents acting on humans as provided by the present invention. Moreover, there has existed no report on a nanodispenser having such precision as to attain spatial control on the micrometer scale and volumes of droplets on the nanoliter scale like a nanodispenser having an automatic dilution function as provided by the present invention for the purpose of fully automating super-multianalyte assays. All previous reports on microdispensers based on adapted inkjet printer heads are directed to a size greater by several orders of magnitude. The combination of state-of-the-art heads and X-Y stage enabled the preparation of the nanodispenser of the present invention. Conventional technology has so far not shown that a rapid super-multianalyte evaluation of drugs/toxic agents can be performed under 500-100,000 different culture conditions on a single preparation by combining a high-density cell array substrate bearing 500-100,000 culture environments each containing a single cell in a completely separated culture medium and a nanodispenser having automatic dilution function as provided by the present invention. Moreover, the present invention also relates to a unique technology in the world especially in that it provides simultaneous super-multianalyte assays suitable for even new drug development requiring high-speed processing of enormous amount of information and environmental impact assessment. The present invention attaches maximum importance to convenience and low running costs as its objects to allow for practical use by physicians in the clinical field. As has been described above, the present invention relates to a very unique technology that has a lot of potential for industrial application far beyond the previous level remaining at the stage of fundamental research.

### EXAMPLES

The following examples further illustrate the present invention without, however, limiting the invention thereto.

### Examples 1-5

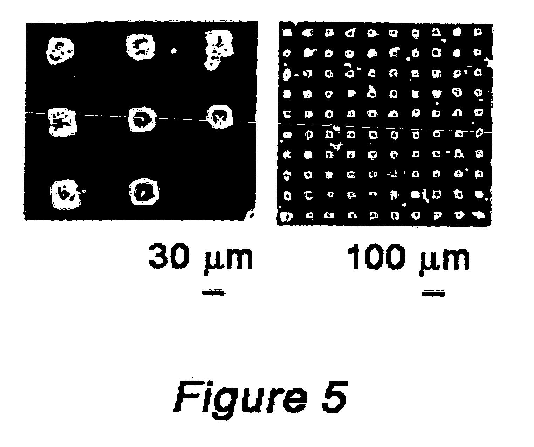

Poly-N-isopropylacrylamide, polyacrylamide and polydimethylsiloxane were used as cell adhesive polymer, hydrophilic polymer and highly hydrophobic polymer, respectively. The concentrations of the monomer and polymer solutions used for preparing each layer are shown in Table 1. These solutions were used for preparing each layer by the procedure described below. First, a solution of N-isopropylacrylamide monomer in isopropyl alcohol at the concentration shown in Table 1 was applied on a polystyrene base in an amount of 0.01 ml/cm² in order to prepare a cell adhesive polymer-coated domain. The coating was irradiated with electron beams at a dose of 0.25 MGy to coat the surface of the base with poly-N-isopropylacrylamide. After irradiation, the substrate was thoroughly washed with water and dried to give a substrate having a poly-N-isopropylacrylamide layer. The amount of poly-N-isopropylacrylamide coated on the substrate is shown in Table 2. Then, a solution of acrylamide monomer in isopropyl alcohol at the concentration shown in Table 1 was applied on the substrate in an amount of 0.01 ml/cm² in order to prepare a cell non-adhesive hydrophilic polymer-coated domain on the preceding layer. The coating was irradiated with electron beams at a dose similar to the value used in the method described above and then washed and dried to give a substrate having a polyacrylamide layer on the poly-N-isopropylacrylamide layer. The amount of polyacrylamide coated on the substrate is shown in Table 2. A solution of polydimethylsiloxane in ethanol at the concentration shown in Table 1 was further applied on the substrate in an amount of 0.01 ml/cm² in order to prepare a cell non-adhesive highly hydrophobic polymer-coated domain on the preceding layer. The coating was irradiated with electron beams at a dose similar to the value used in the method described above and then washed and dried to give a substrate having a polydimethylsiloxane layer on the preformed substrate. The amount of polydimethylsiloxane coated on the substrate is shown in Table 2. The resulting substrate was placed on a computer-controlled X-Y stage and ablated with an ArF excimer laser (pulsed laser at a wavelength of 193 nm, L5910 manufactured by Hamamatsu Photonics K.K.) so that each layer appeared on the substrate surface. Specifically, the laser was used at a dose of 0.1 J/cm² to expose the polyacrylamide layer. The surface was further ablated with the laser at a dose of 0.1 J/cm² to expose about a 30 µm square of the poly-N-isopropylacrylamide layer at the center of the exposed layer. These processes were repeated to expose 10,000 domains/cm² of the poly-N-isopropylacrylamide layer. Observation under a reflective laser confocal microscope showed that the ablated surfaces were very flat with sharp edges and that each layer had a thickness of about 30 nm as a result of ablation. Elementary analysis of the substrate surface by ESCA showed that the Si element was not present in the polyacrylamide layer and that the N element content in the domains consisting of the poly-N-isopropylacrylamide layer agreed with the content in poly-N-isopropylacrylamide, confirming that each layer could be exposed as the surface of the substrate by the process of this example. Then, walls were formed on the resulting substrate with silicone rubber, in which rat primary hepatic solid tumor cells dispersed at a concentration of 10⁵ cells/ml in a culture medium consisting of Williams E medium containing 5% fetal calf serum (FCS), 10⁻⁸ M dexamethasone, 10⁻⁷ M insulin, 10 mM nicotinamide, and 10 ng/ml epithelial cell growth factor (FGF) were seeded. The substrate was allowed to stand in a 5% carbon dioxide atmosphere at 37 °C for 3 hours, whereby a single rat primary hepatic solid tumor cell adhered to each poly-N-isopropylacrylamide layer. As an example, the appearance of hepatic solid tumor cells on the high-density cell array substrate of Example 1 is shown in Figure 5. The cells recognized the adhesive polymer domains and selectively adhered to them. Then, the culture medium on the substrate was gently aspirated and a dispersion of 0-4.5 mM dichlorobenzene in 10 pl of the culture medium was deposited on each hydrophilic polymer-coated domain at 37°C using a microdispenser consisting of adapted inkjet printer heads (10 concentrations in steps of 0.5 mM for each group of 1000 hydrophilic polymer-coated domains). After 10 minutes, cells in the culture medium in which 2.5 mM or more dichlorobenzene were dispersed were morphologically altered. Then, the entire substrate was thoroughly washed with the culture medium and finally the culture medium was gently aspirated again so that the culture medium remained only in each hydrophilic polymer-coated domain. The substrate was allowed to cool at 20°C for 15 minutes, after which cells on the point of falling off the substrate surface could be separated and recovered for each dichlorobenzene concentration using a syringe.

### Comparative examples 1-4

High-density cell array substrates were obtained by a similar preparation process to that of Examples 1-5 except that the solution concentrations shown in Table 1 were used. Then, cells were seeded and attempts were made to allow the culture medium to remain only in each hydrophilic polymer-coated domain by similar procedures. The coating amounts on the resulting substrates are shown in Table 2. It was difficult to retain the culture medium when the coating amount of the hydrophilic polymer was small. When it was excessive, the hydrated polymer layer became considerably irregular to affect the visual field during microscopic observation so that it was unsuitable as a substrate. It was difficult to repel the culture medium when the coating amount of the highly hydrophobic polymer was too small. When it was excessive, the polymer layer became considerably irregular to affect the visual field during microscopic observation so that it was unsuitable as a substrate.

**Table 1**

| Solution concentrations | | | |
|---|---|---|---|
| | N-Isopropylacrylamide | Acrylamide | Polydimethylsiloxane |
| Example 1 | 30 wt% | 20 wt% | 30 wt% |
| Example 2 | 30 wt% | 30 wt% | 20 wt% |
| Example 3 | 40 wt% | 20 wt% | 30 wt% |
| Example 4 | 40 wt% | 30 wt% | 20 wt% |
| Example 5 | 50 wt% | 20 wt% | 20 wt% |
| Comparative example 1 | 30 wt% | 1 wt% | 20 wt% |
| Comparative example 2 | 30 wt% | 60 wt% | 20 wt% |
| Comparative example 3 | 30 wt% | 20 wt% | 1 wt% |
| Comparative example 4 | 30 wt% | 20 wt% | 55 wt% |

**Table2**

| Coating amounts | | | |
|---|---|---|---|
| | Poly-N-isopropylacrylamide | Polyacrylamide | Polydimethylsiloxane |
| Example 1 | 1.5 µg/cm² | 1.2 µg/cm² | 1.0 µg/cm² |
| Example 2 | 1.6 µg/cm² | 2.0 µg/cm² | 0.8 µg/cm² |
| Example 3 | 1.8 µg/cm² | 1.3 µg/cm² | 1.1 µg/cm² |
| Example 4 | 1.7 µg/cm² | 2.1 µg/cm² | 0.9 µg/cm² |
| Example | 2.0 µg/cm² | 1.3 µg/cm² | 0.9 µg/cm² |
| Comparative example 1 | 1.4 µg/cm² | 0.05 µg/cm² | 1. 0 µg/cm² |
| Comparative example 2 | 1. 5 µg/cm² | 21.0 µg/cm² | 0.9 µg/cm² |
| Comparative example 3 | 1.5 µg/cm² | 1.2 µg/cm² | 0.05 µg/cm² |
| Comparative example 4 | 1.4 µg/cm² | 1.2 µg/cm² | 25.0 µg/cm² |

### Example 6

A high-density cell array substrate was obtained by a similar preparation process to that of Example 1 except that a 20 wt% solution of a phenol resin having 1,2-naphthoquinone diazide-4-sulfonate on the side chain in dioxane was used to prepare cell adhesive polymer-coated domains. The amount of said polymer coated on the resulting substrate was 1.1 µg/cm². Then, cells were seeded and an assay was made with dichlorobenzene by similar procedures. Cells could be separated and recovered by irradiating each hydrophilic polymer-coated domain with UV light for 5 minutes. It could be confirmed that this method could selectively separate and recover cells in a single hydrophilic polymer-coated domain by focusing light on each hydrophilic polymer-coated domain.

### INDUSTRIAL APPLICABILITY

High-density cell array substrates of the present invention and assay systems using them can greatly reduce side effects of drugs and dramatically improve the performance of drug therapy for diseases by optimizing the type and concentration of the drug used (e.g., anticancer agent). This would largely contribute to medical economy by improving the prognoses of patients and reducing recurrence. The present invention provides a very useful technology that can also be used for drug development, environmental impact assessment and basic life science researches.

## Claims

1. A substrate for a high-density cell array **characterized in that** it has a surface comprising an ordered array of discrete microdomains coated with a cell adhesive polymer and successively surrounded by a domain coated with a cell non-adhesive hydrophilic polymer and then a domain coated with a cell non-adhesive highly hydrophobic material.

2. The substrate for a high-density cell array of claim 1 **characterized in that** it comprises an array of 500-100,000 domains coated with a cell adhesive polymer per cm².

3. The substrate for a high-density cell array of claim 1 or 2 **characterized in that** the cell adhesive polymer is a stimulus-responsive polymer.

4. The substrate for a high-density cell array of claim 3 **characterized in that** the stimulus-responsive polymer is photoresponsive.

5. The substrate for a high-density cell array of claim 1 **characterized in that** each domain coated with a cell non-adhesive hydrophilic polymer surrounds one domain coated with a cell adhesive polymer.

6. A process for preparing the substrate for a high-density cell array of any one of claims 1 to 5, comprising providing a stack of at least a cell adhesive polymer layer, a cell non-adhesive hydrophilic polymer layer and a cell non-adhesive highly hydrophobic material layer on a base and subjecting it to laser ablation in such a manner that each layer partially appears as a part of the surface of the substrate.

7. The process for preparing the substrate for a high-density cell array of claim 6 **characterized in that** the stack comprises a stimulus-responsive polymer layer, a hydrophilic polymer layer and a highly hydrophobic polymer layer in this order from the base side.

8. A method for using the substrate for a high-density cell array of any one of claims 1 to 5 comprising adhering a cell to each domain coated with a cell adhesive polymer and depositing a small amount of a culture medium on each domain coated with a cell non-adhesive hydrophilic polymer.

9. The method for using the substrate for a high-density cell array of claim 8 comprising introducing various concentrations of a chemical or various kinds of chemicals into each cell cultured in the microdomains using an automatic chemical dispenser based on inkjet printer heads optionally having dilution function, and then determining the viability of each cell or measuring the activity.

10. The method for using the substrate for a high-density cell array of claim 8 comprising transferring a specific gene into each cell cultured in the microdomains, and then measuring the activity of each cell.

11. A method for using the substrate for a high-density cell array of any one of claims 8 to 10 comprising separating and recovering only cells having a specific activity by giving a stimulus to the domain coated with a cell adhesive polymer to which they adhere after cell evaluation or genetic manipulation.

12. The method for using the substrate for a high-density cell array of claim 11 **characterized in that** the stimulus given to the domain coated with a cell adhesive polymer is light.

13. A screening method of a chemical, comprising using the substrate for a high-density cell array of any one of claims 1 to 5.

14. The screening method of claim 13 wherein the chemical is a medicine.

15. The screening method of claim 14 wherein the medicine is an anticancer agent.

16. The screening method of claim 13 wherein the chemical is a toxic agent or an environmentally hazardous substance.

17. A gene transfer method comprising using the substrate for a high-density cell array of any one of claims 1 to 5.

## Patentansprüche

1. Substrat für ein Zell-Array hoher Dichte, **dadurch gekennzeichnet, dass** es eine Oberfläche hat, die ein geordnetes Array eigenständiger Mikrodomänen umfasst, beschichtet mit einem Zell-adhäsiven Polymer und nacheinander folgend von einer Domäne umgeben, die mit einem nicht-Zell-adhäsiven hydrophilen Polymer beschichtet ist, und dann von einer Domäne umgeben, die mit einem nicht-Zell-adhäsiven hochgradig hydrophoben Material beschichtet ist.

2. Substrat für ein Zell-Array hoher Dichte gemäß Anspruch 1, **dadurch gekennzeichnet, dass** es ein Array von 500-100.000 Domänen pro cm² umfasst, beschichtet mit einem Zell-adhäsiven Polymer.

3. Substrat für ein Zell-Array hoher Dichte gemäß Anspruch 1 oder 2, **dadurch gekennzeichnet, dass** das Zell-adhäsive Polymer ein auf Reiz reagierendes Polymer ist.

4. Substrat für ein Zell-Array hoher Dichte gemäß Anspruch 3, **dadurch gekennzeichnet, dass** das auf Reiz reagierende Polymer auf Licht reagiert.

5. Substrat für ein Zell-Array hoher Dichte gemäß Anspruch 1, **dadurch gekennzeichnet, dass** jede Domäne, die mit einem nicht-Zell-adhäsiven hydrophilen Polymer beschichtet ist, eine Domäne umgibt, die mit einem Zell-adhäsiven Polymer beschichtet ist.

6. Verfahren zur Herstellung des Substrats für ein Zell-Array hoher Dichte gemäß mindestens einem der Ansprüche 1 bis 5, umfassend die Bereitstellung eines Stapels von mindestens einer Schicht Zell-adhäsiven Polymers, einer Schicht nicht-Zell-adhäsiven hydrophilen Polymers und einer Schicht nicht-Zell-adhäsiven hochgradig hydrophoben Materials auf einer Basis, und das Unterziehen desselben einer Laser-Abtragung in solch einer Weise, dass jede Schicht teilweise als ein Teil der Oberfläche des Substrats erscheint.

7. Verfahren zur Herstellung des Substrats für ein Zell-Array hoher Dichte gemäß Anspruch 6, **dadurch gekennzeichnet, dass** der Stapel eine Schicht auf Reiz reagierenden Polymers, eine Schicht hydrophilen Polymers und eine Schicht hochgradig hydrophoben Polymers in dieser Reihenfolge von der Seite der Basis her umfasst.

8. Verfahren zur Verwendung des Substrats für ein Zell-Array hoher Dichte gemäß mindestens einem der Ansprüche 1 bis 5, umfassend das Adhärieren einer Zelle auf jede Domäne, die mit einem Zell-adhäsiven Polymer beschichtet ist, und das Aufbringen einer kleinen Menge eines Kulturmediums auf jede Domäne, die mit einem nicht-Zell-adhäsiven hydrophilen Polymer beschichtet ist.

9. Verfahren zur Verwendung des Substrats für ein Zell-Array hoher Dichte gemäß Anspruch 8, umfassend das Einbringen verschiedener Konzentrationen einer Chemikalie oder verschiedener Arten von Chemikalien in jede Zelle, die in den Mikrodomänen kultiviert wird, mittels eines automatischen Chemikalien-Verteilers, der auf Tintenstrahldruckerköpfen basiert, optional mit Verdünnungsfunktion, und dann das Bestimmen der Viabilität jeder Zelle oder die Messung der Aktivität.

10. Verfahren zur Verwendung des Substrats für ein Zell-Array hoher Dichte gemäß Anspruch 8, umfassend die Übertragung eines spezifischen Gens in jede Zelle, die in den Mikrodomänen kultiviert wird, und dann die Messung der Aktivität jeder Zelle.

11. Verfahren zur Verwendung des Substrats für ein Zell-Array hoher Dichte gemäß mindestens einem der Ansprüche 8 bis 10, umfassend das Abtrennen und Gewinnen nur von Zellen mit einer spezifischen Aktivität, indem, nach der Evaluierung oder genetischen Manipulation der Zelle, der Domäne, die mit einem Zell-adhäsiven Polymer beschichtet ist, an welches sie adhärieren, ein Reiz gegeben wird.

12. Verfahren zur Verwendung des Substrats für ein Zell-Array hoher Dichte gemäß Anspruch 11, **dadurch gekennzeichnet, dass** der Reiz, der der Domäne gegeben wird, die mit einem Zell-adhäsiven Polymer beschichtet ist, Licht ist.

13. Screening-Verfahren einer Chemikalie, umfassend die Verwendung des Substrats für ein Zell-Array hoher Dichte gemäß mindestens einem der Ansprüche 1 bis 5.

14. Screening-Verfahren gemäß Anspruch 13, wobei die Chemikalie eine Medizin ist.

15. Screening-Verfahren gemäß Anspruch 14, wobei die Medizin ein Anti-Krebs-Mittel ist.

16. Screening-Verfahren gemäß Anspruch 13, wobei die Chemikalie ein toxisches Mittel oder eine für die Umwelt gefährliche Substanz ist.

17. Gentransfer-Verfahren, umfassend die Verwendung des Substrats für ein Zell-Array hoher Dichte gemäß mindestens einem der Ansprüche 1 bis 5.

## Revendications

1. Substrat pour un réseau de cellules à haute densité, **caractérisé en ce qu'**il présente une surface comprenant un réseau ordonné de microdomaines discrets revêtus d'un polymère d'adhésion cellulaire et successivement entourés d'un domaine revêtu d'un polymère hydrophile n'adhérant pas aux cellules, puis d'un domaine revêtu d'un matériau hautement hydrophobe n'adhérant pas aux cellules.

2. Substrat pour un réseau de cellules à haute densité selon la revendication 1, **caractérisé en ce qu'**il comprend un réseau de 500 à 100 000 domaines revêtus d'un polymère d'adhésion cellulaire par cm².

3. Substrat pour un réseau de cellules à haute densité selon la revendication 1 ou 2, **caractérisé en ce que** le polymère d'adhésion cellulaire est un polymère réagissant aux stimuli.

4. Substrat pour un réseau de cellules à haute densité selon la revendication 3, **caractérisé en ce que** le polymère réagissant aux stimuli est photosensible.

5. Substrat pour un réseau de cellules à haute densité selon la revendication 1, **caractérisé en ce que** chaque domaine revêtu d'un polymère hydrophile n'adhérant pas aux cellules entoure un domaine revêtu d'un polymère d'adhésion cellulaire.

6. Procédé de préparation du substrat pour un réseau de cellules à haute densité selon l'une quelconque des revendications 1 à 5, comprenant la fourniture d'un empilement d'au moins une couche de polymère d'adhésion cellulaire, une couche de polymère hydrophile n'adhérant pas aux cellules et une couche de matériau hautement hydrophobe n'adhérant pas aux cellules sur une base, et l'étape consistant à le soumettre à une ablation par laser, de façon à ce que chaque couche apparaisse partiellement en tant que partie de la surface du substrat.

7. Procédé de préparation du substrat pour un réseau de cellules à haute densité selon la revendication 6, **caractérisé en ce que** l'empilement comprend une couche de polymère réagissant aux stimuli, une couche de polymère hydrophile et une couche de polymère hautement hydrophobe, dans cet ordre à partir de la base.

8. Procédé d'utilisation du substrat pour un réseau de cellules à haute densité selon l'une quelconque des revendications 1 à 5, comprenant l'étape consistant à faire adhérer une cellule à chaque domaine revêtu d'un polymère d'adhésion cellulaire et l'étape consistant à déposer une petite quantité d'un milieu de culture sur chaque domaine revêtu d'un polymère hydrophile n'adhérant pas aux cellules.

9. Procédé d'utilisation du substrat pour un réseau de cellules à haute densité selon la revendication 8, comprenant l'introduction de diverses concentrations d'une substance chimique ou de divers types de substances chimiques dans chaque cellule cultivée dans les microdomaines, en utilisant un distributeur automatique de substances chimiques basé sur des têtes d'impression à jet d'encre comportant éventuellement une fonction de dilution, puis la détermination de la viabilité de chaque cellule ou la mesure de l'activité.

10. Procédé d'utilisation du substrat pour un réseau de cellules à haute densité selon la revendication 8, comprenant le transfert d'un gène spécifique dans chaque cellule cultivée dans les microdomaines, puis la mesure de l'activité de chaque cellule.

11. Procédé d'utilisation du substrat pour un réseau de cellules à haute densité selon l'une quelconque des revendications 8 à 10, comprenant la séparation et la récupération des seules cellules ayant une activité spécifique, en envoyant un stimulus sur le domaine revêtu d'un polymère d'adhésion cellulaire sur lequel elles adhèrent après l'évaluation cellulaire ou la manipulation génétique.

12. Procédé d'utilisation du substrat pour un réseau de cellules à haute densité selon la revendication 11, **caractérisé en ce que** le stimulus envoyé sur le domaine revêtu d'un polymère d'adhésion cellulaire est la lumière.

13. Procédé de criblage d'une substance chimique, comprenant l'utilisation du substrat pour un réseau de cellules à haute densité selon l'une quelconque des revendications 1 à 5.

14. Procédé de criblage selon la revendication 13, dans lequel la substance chimique est un médicament.

15. Procédé de criblage selon la revendication 14, dans lequel le médicament est un agent anticancéreux.

16. Procédé de criblage selon la revendication 13, dans lequel la substance chimique est un agent toxique ou une substance nuisible pour l'environnement.

17. Méthode de transfert de gènes comprenant l'utilisation du substrat pour un réseau de cellules à haute densité selon l'une quelconque des revendications 1 à 5.
